# EUROPEAN PATENT APPLICATION

(11) **EP 2 014 660 A2**
(43) Date of publication of application: **14.01.2009**
(21) Application number: 08018872.5
(22) Date of filing: 02.06.2004
(51) Int. Cl.: C07D 473/18

(54) **Novel crystalline forms of valacyclovir hydrochloride**

(30) Priority: 02.06.2003 US 475581 P
(62) Divisional of application: 04754365.7
(71) Applicant: Teva Pharmaceutical Industries Ltd, 49131 Petah Tiqva (IL)
(72) Inventor: Lifshitz, Igor, 49602 Petah-Tiqva (IL); Wizel, Shlomit, 49742 Petah Tikva (IL); Frenkel, Gustavo, 84841 Beer Sheva (IL); Kor, Ilan, Shoham (IL)
(74) Representative: Gallagher, Kirk James

(57) **Abstract**

Provided are novel crystalline forms of valacyclovir hydrochloride denominated forms VIII, IX, X, XI, and XII and methods of making them. Also provided is a novel method for making valacyclovir hydrochloride in crystalline form V.

## Description

### FIELD OF THE INVENTION

The present invention relates to novel crystalline forms (polymorphs and pseudopolymorphs) of the antiviral compound valacyclovir hydrochloride, and methods for obtaining them.

### RELATED APPLICATION

This application claims the benefit of the June 2, 2003 filing date of United States Provisional Patent Application 60/475,581.

### BACKGROUND

Valacyclovir is an L-valyl ester prodrug of acyclovir. Acyclovir is an analog of a natural nucleoside which has been found to have high anti-viral activity. Acyclovir is widely used in the treatment and prophylaxis of viral infections in humans, particularly infections caused by the herpes group of viruses. *See* Goodman and Gilman's, The Pharmacological Basis of Therapeutics 1193-1198 (9th ed. 1996).

Acyclovir is an acyclic guanine nucleoside analog that lacks a 3'-hydroxyl on the side chain. Acyclovir has the chemical name 6H-Purin-6-one, 2-amino-1,9-dihydro-9-[(2-hydroxyethoxy)methyl]. (CAS Registry No. 59277-89-3.) Acyclovir as the sodium salt is currently marketed as ZOVIRAX^{®}. The chemical structure of acyclovir is shown as Formula I.

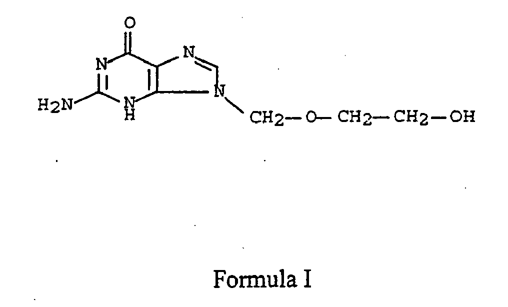

Valacyclovir has the chemical name 1-valine, 2-[(2-amino-1,6-dihydro-6-oxo -9H-purin-9-yl)methoxy]ethyl ester. (CAS Registry No. 124832-26-4.) Valacyclovir is currently marketed as VALTREX^{®}. The chemical structure of valacyclovir is shown as Formula II.

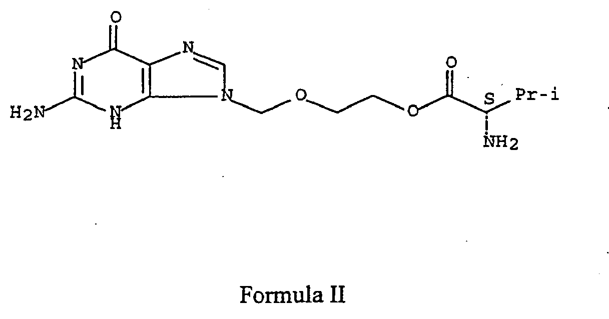

For oral administration, it may be advantageous to administer valacyclovir rather than acyclovir because acyclovir is reportedly poorly absorbed from the gastrointestinal tract after oral administration in both animals and humans. In contrast, valacyclovir is rapidly absorbed from the gastrointestinal tract after oral administration. Moreover, valacyclovir is converted rapidly and virtually completely to acyclovir after oral administration in healthy adults. The conversion of valacyclovir is thought to result from first-pass intestinal and hepatic metabolism through enzymatic hydrolysis.

Acyclovir is said to kill viruses by inhibiting viral DNA synthesis. Because acyclovir is a guanosine analog which lacks the 3'-hydroxyl on the side chain, it causes DNA chain termination during viral DNA replication. In virus infected cells, acyclovir is converted to the monophosphate derivative (acyclovir-MP) by a viral enzyme, thymidinine kinase. Acyclovir-MP is then phosphorylated to the diphosphate and triphosphate analogs by cellular enzyme. Incorporation of activated acyclovir into the primer strand during viral DNA replication, leads to chain termination, since without the 3' hydroxyl the DNA chain can not be extended. Since uninfected cells lack the viral enzyme thymidine kinase, acyclovir is selectively activated only in cells infected with viruses that code for the appropriate kinases.

U.S. Pat. No. 4,199,574 discloses the treatment of viral infections with acyclovir.

U.S. Pat. No. 4,957,924 (the '924 Patent") discloses amino acid esters of the purine nucleoside acyclovir, pharmaceutically acceptable salts thereof and their use in the treatment of herpes virus infections. Also disclosed are pharmaceutical formulations and processes for the preparation of such compounds. Valacyclovir and its salts, including the hydrochloride salt, are among the disclosed compounds.

The '924 patent further discloses a method for the preparation of valacyclovir by condensation of CBZ-Valine and acyclovir in Dimethylformamide (DMF) with catalytic amount of 4-dimethylaminopyridine (DMAP) and Dicyclohexylcarbodiimide (DCC) as a coupling reagent.

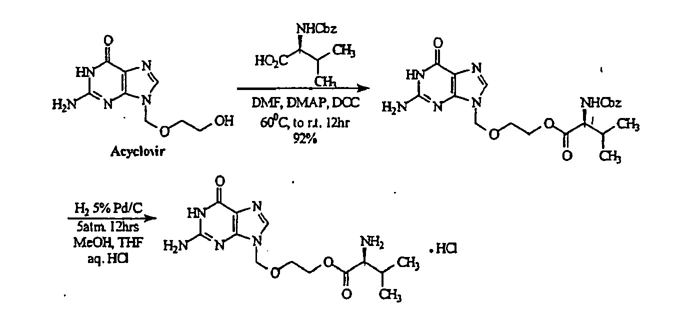

U.S. Pat. No. 6,107,302, incorporated herein by reference, discloses an anhydrous crystalline form of valacyclovir hydrochloride and a process of preparation.

The discovery of a new crystalline form of a pharmaceutically useful compound provides an opportunity to improve the performance characteristics of a pharmaceutical product. It enlarges the repertoire of materials that a formulation scientist has available for designing, for example, a pharmaceutical dosage form of a drug with a targeted release profile or other desired characteristic. It is clearly advantageous when this repertoire is enlarged by the discovery of new crystalline forms of a useful compound. For a general review of polymorphs and the pharmaceutical applications of polymorphs see G.M. Wall, Pharm Manuf. 3, 33 (1986); J.K. Haleblian and W. McCrone, J. Pharm. Sci., 58, 911 (1969); and J.K. Haleblian, J. Pharm. Sci., 64, 1269 (1975), all of which are incorporated herein by reference.

The solid state physical properties of crystalline forms of a pharmaceutically useful hydrochloride salt can be influenced by controlling the conditions under which the hydrochloride salt is obtained in solid form. Solid state physical properties include, for example, the flowability of the milled solid. Flowability affects the ease with which the material is handled during processing into a pharmaceutical product. When particles of the powdered compound do not flow past each other easily, a formulation specialist must take that fact into account in developing a tablet or capsule formulation, which may necessitate the use of glidants such as colloidal silicon dioxide, talc, starch or tribasic calcium phosphate.

Another important solid state property of a pharmaceutical compound is its rate of dissolution in aqueous fluid. The rate of dissolution of an active ingredient in a patient's stomach fluid can have therapeutic consequences since it imposes an upper limit on the rate at which an orally-administered active ingredient can reach the patient's bloodstream. The rate of dissolution is also a consideration in formulating syrups, elixirs and other liquid medicaments. The solid state form of a compound may also affect its behavior on compaction and its storage stability.

These practical physical characteristics are influenced by the conformation and orientation of molecules in the unit cell, which defines a particular crystalline form of a substance. The crystalline form may give rise to thermal behavior different from that of the amorphous material or another crystalline form. Thermal behavior is measured in the laboratory by such techniques as capillary melting point, thermogravimetric analysis (TGA) and differential scanning calorimetry (DSC) and can be used to distinguish some crystalline forms from others. A particular crystalline form may also give rise to distinct spectroscopic properties that may be detectable by powder X-ray crystallography, solid state ¹³C NMR spectrometry and infrared spectrometry.

### SUMMARY OF THE INVENTION

In one aspect, the present invention relates to valacyclovir hydrochloride in crystalline forms VIII, IX, X, XI, and XII, as well as admixtures of two or more of these forms.

In another aspect, the present invention relates to methods of making forms VIII, IX, X, XI, and XII, and mixtures thereof. The present invention also relates to pharmaceutical compositions containing valacyclovir hydrochloride in crystalline forms VIII, IX, X, XI, and XII, as well as mixtures of two or more of these.

In one aspect, the present invention, relates to a crystalline form of valacyclovir hydrochloride, denominated, form VIII, which can be characterized by x-ray diffraction reflections at 7.1°, 10.7°, 13.2°, 14.2°, 21.4°, 21.8°, and 25.0° ± 0.2° 2θ, or by DSC endotherms at about 60°C, 100°C, and 133°C.

In a another aspect, the present invention relates to a crystalline form of valacyclovir hydrochloride, denominated form IX, which can be characterized by either x-ray diffraction reflections at 8.7°, 10:5°, 24.1°, 26.3° and 27.0° ± 0.2° 2θ, or a DSC endotherm peak at about 67°C.

In a further aspect, the present invention relates to a crystalline form of valacyclovir hydrochloride, denominated form X, which can be characterized by x-ray diffraction reflections at 6.7°, 7.0°, 17.5°, 21.0°, 22.4°, 24.5°, and 31.6°± 0.2° 2θ, or DSC endotherms at about 55° C, 75° C, 95° C, and 133° C.

In still yet another aspect, the present invention relates to a crystalline form of valacyclovir hydrochloride, denominated form XI, which can be characterized by x-ray reflections at about 14.4°, 21.6°, and 25.2° ± 0.2° 2θ, or DSC endotherms at about 49°, 107°, 132°, and 175°C. Valacyclovir hydrochloride form XI can be further characterized as having a water content of about 6% to about 8% as determined by Karl Fisher analysis.

In yet another aspect, the present invention relates to a crystalline form of valacyclovir hydrochloride, denominated form XII, which can be characterized by x-ray reflections at about 7.2°, 11.9°, 27.4°, and 28.1° ± 0.2° 2θ, or by a multi-endothermic DSC thermogram having minima at about 135° and 155° C and a peak at about 177° C.

In yet another aspect, the present invention relates to a method of making valacyclovir hydrochloride in form V including the steps of: providing, at reflux, an approximately 25% (w/v) solution of valacyclovir hydrochloride in a mixture of water and methanol, 1:15 (v:v); cooling the solution to 20° C at a cooling rate of about 10 degrees per hour; maintaining the resulting slurry at 20°C for a holding time; and isolating crystalline valacyclovir hydrochloride form V.

In still a further aspect, the present invention relates to a method of making valacyclovir hydrochloride form V including the steps of: providing an approximately 5% (w/v) solution of valacyclovir hydrochloride in a mixture of acetone and methanol, 1:3 (v:v), at a temperature of about 54° C; cooling the solution to about -5° C or less at a cooling rate of about 10 degrees per hour; maintaining the resulting slurry at -5° C or less for a holding time; and isolating valacyclovir hydrochloride form V.

In another aspect, the present invention relates to a method of making valacyclovir hydrochloride form VIII including the steps of providing a solution of at least about 15% (w/v) concentration of valacyclovir hydrochloride in a first solvent wherein the first solvent comprises at least about 50% (v/v) isopropailol, especially about 55%, the remainder consisting essentially of water, at an initial temperature below about 60°C, especially about 50°C, and combining the solution with a second solvent consisting essentially of isopropanol.

In a further aspect, the present invention provides a method of making valacyclovir hydrochloride form VIII including the steps of providing a solution of valacyclovir hydrochloride in a crystallization solvent that is a mixtures of first solvent that consists essentially of water and a second solvent that consists essentially of isopropanol at an initial temperature; especially about 37°C; cooling the solution provided to a seeding temperature, especially about 35°C; seeding the solution provided with valacyclovir hydrochloride; maintaining the seeded solution at the seeding temperature for a period of time from about 2 to about 4 hours, whereby a suspension is formed; cooling the suspension to a temperature of about -5° C or less; and isolating the crystalline valacyclovir hydrochloride VIII.

In another aspect, the present invention relates to a method of making crystalline valacyclovir hydrochloride crystalline form IX including the steps of providing a solution of less than about 10% (w/v) concentration of valacyclovir hydrochloride in a first solvent that is a 18/82 v/v mixture of essentially water and a second solvent that consists essentially of isopropanol at an initial temperature of at least about 60° C; cooling the solution to a temperature of about 59°C and maintaining the solution at that temperature at least until the solution becomes turbid; cooling the turbid solution to a temperature of -5° C or below at a cooling rate of about 4 to about 6 degrees per minute; and isolating the crystalline valacyclovir hydrochloride form IX.

In a further aspect, the present invention relates to a method of making crystalline valacyclovir hydrochloride form X including the steps of: providing, at a temperature of about 30°C to about 35°C, a solution of at least about 20% concentration, w/v, of valacyclovir hydrochloride in a first solvent that consists essentially of water; combining the solution with a first portion of second solvent, equal to about 2 to about 2.3 times the volume of solution, that consists essentially of ethanol, and maintaining the combination at a temperature of about 30°C to about 35°C for about 1 to about 3 hours; further combining the combination with a second portion of second solvent, equal to about 2 to about 2.3 times the initial volume of the solution provided; cooling the combination so obtained to a temperature of about -5°C or less; and isolating the crystalline valacyclovir hydrochloride form X.

In yet a further aspect, the present invention relates to a method of making crystalline valacyclovir hydrochloride form V including the steps of: providing, at reflux, an approximately 25% (w/v) solution of valacyclovir hydrochloride in a mixture of water and methanol, 1:15 (v:v); cooling the solution to 20° C at a cooling rate of about 10 degrees per hour; maintaining the resulting slurry at 20°C for a holding time; and isolating crystalline valacyclovir hydrochloride form V.

In another aspect, the present invention relates to a pharmaceutical composition including any one of valacyclovir hydrochloride in form VIII, IX, X, XI, or XII, or a mixture of any of them, with at least one pharmaceutically acceptable excipient.

### BRIEF DESCRIPTION OF THE DRAWINGS

Figure 1 is a typical powder x-ray diffraction diagram of valacyclovir hydrochloride form V.
Figure 2 is a typical powder x-ray diffraction diagram of valacyclovir hydrochloride form VIII.
Figure 3 is a typical DSC thermogram of valacyclovir hydrochloride form VIII.
Figure 4 is a typical powder x-ray diffraction diagram of valacyclovir hydrochloride form IX.
Figure 5 is a typical DSC thermogram of valacyclovir hydrochloride form IX.
Figure 6 is a typical powder x-ray diffraction diagram of valacyclovir hydrochloride form X.
Figure 7 is a typical DSC thermogram of valacyclovir hydrochloride form X.
Figure 8 is a typical powder x-ray diffraction diagram of valacyclovir hydrochloride form XI.
Figure 9 is a typical DSC thermogram of valacyclovir hydrochloride form XI.
Figure 10 is a typical powder x-ray diffraction diagram of valacyclovir hydrochloride form XII.
Figure 11 is a typical DSC thermogram of valacyclovir hydrochloride form XII.

### DETAILED DESCRIPTION OF THE INVENTION

The present invention provides valacyclovir hydrochloride in new crystalline forms VII, IX, X, XI, and XII as well as admixtures of two or more of these forms. The present invention also provides methods for preparing valacyclovir hydrochloride in crystalline formes VII, IX, X, XI, and XII, as well as admixtures of two or more of these forms.

The present invention further relates to the solid state physical properties of these crystalline forms of valacyclovir hydrochloride as prepared by any of the methods of the present invention, as well as by other methods known to those skilled in the art.

As used herein, unless the context requires otherwise, the term "valacyclovir hydrochloride" includes anhydrous forms; hydrates, solvates, and all crystalline forms (both polymorphs and pseudopolymorphs), of valacyclovir hydrochloride.

As used herein in connection with a measured quantity, the term "about" refers to that variation in the measured quantity as would be expected by the skilled artisan performing the measurement and exercising a level of care commensurate with the objective of the measurement and the precision of the measuring apparatus being used.

For the purposes of this specification, ambient or room temperature is from about 20°C to about 25°C, elevated temperature means above about 38°C, and cold temperature means below about -10°C.

All powder x-ray diffraction patterns were obtained by methods known in the art using a Scintag X'TRA X-ray powder diffractometer, equipped with a solid state Si(Li) detector thermoelectrically cooled, at scanning speed of 3° min.⁻¹. The scanning range was 2-40 degrees two-theta. Copper radiation of λ = 1.5418° was used. The term x-ray diffraction "peaks" as used herein refers to x-ray diffraction reflections measured using an x-ray powder diffractometer. "Wet" samples (i.e. samples not dried) were analyzed "as is". Dry samples were gently ground before analysis.

The differential thermal analysis ("DTA") and thermogravimetric analysis ("TGA") curves presented herein were obtained by methods known in the art using a DTG Shimadzu model DTG-50 (combined TGA and DTA). The weight of the samples was about 9 to about 13 mg. The samples were scanned up to about 300°C at a rate of 10°C/min. Sample chambers were purged with nitrogen gas at a flow rate of 20 mL/min. Uncovered standard alumina crucibles were used.

Karl Fisher analysis, which is well known in the art, is also used to determine the quantity of water in a sample.

The term "water content" refers to the content of water based upon the Loss on Drying method (the "LOD" method) as described in U.S. Pharmacopeia Forum, Vol. 24, No. 1, p. 5438 (Jan - Feb 1998), the Karl Fisher assay for determining water content or thermogravimetric analysis (TGA). The term "equivalents of water" means molar equivalents of water. All percentages referenced herein are by weight unless otherwise indicated.

Those skilled in the art will also understand that the term "anhydrous" when used in reference to valacyclovir hydrochloride describes valacyclovir hydrochloride which is substantially free of water. Those skilled in the art will appreciate that the term "hydrate" when used in reference to valacyclovir hydrochloride describes a crystalline material having a water content of about 6 - 10 % w/w.

As used herein in connection with a mixture of liquids, vol-%, %v, v/v, or v:v refer to the ratio of volumes of liquids used to make the mixture. Thus, 50% (v/v) refers to a mixture of approximately equal volumes of two liquids. Similarly, in connection with a mixture of liquids, v/v refers to the ratio of volumes of liquids used to make the mixture. For example, a mixture of 50 mL of A and 150 mL B can be described as a 1:3 3 mixture, v/v, of A and B, or, alternatively, 25 vol-% A.

In one embodiment, the present invention provides a method of making valacyclovir hydrochloride in form V.

Valacyclovir hydrochloride in form V is characterized by x-ray reflections (peaks) at about 6.7°, 15.7°, 16.2°, and 22.6° ± 0.2 degrees two-theta.

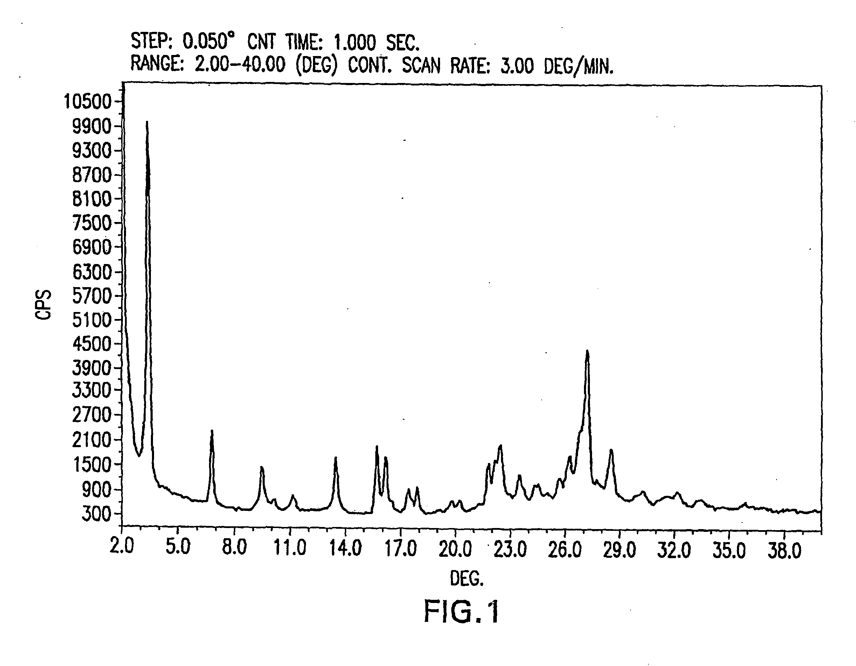

Valacyclovir hydrochloride in form V of the present invention can be further characterized by additional x-ray reflections (peaks) at about 3.4°, 9.5°, 13.5°, 21.9°, 27.2°, and 28.6°± 0.2° two-theta. Figure 1 shows a representative x-ray powder diffraction pattern of valacyclovir hydrochloride in form V.

Valacyclovir for V is described in co-pending United States Patent Application 10/236,729.

In yet another embodiment, the present invention relates to transformation, especially by drying or thermal treatment, of herein disclosed crystalline forms of valacyclovir hydrochloride to valacyclovir hydrochloride form I.

form I can be characterized by x-ray diffraction peaks(reflections) at about 3.7, 8.6, , 10.6, 10.9, 16.5, 24.0, and 27.2 ± 0.2 degrees two-theta, and further characterized by x-ray diffraction peaks (reflections) at 9.5, 13.3, 20.1, 21.4, and 26.7 degrees two theta. Form I exhibits a weight loss of between about 6% and about 9% as measured by thermogravimetric analysis over the temperature range between about 25°C and about 140°C. This water content corresponds to the stochiometric amount of water in the sesquihydrate and is agreement with water contend determined by Karl-Fisher analysis.

In a further embodiment, the present invention relates to transformation, especially by drying or squeezing, of herein disclosed crystalline forms of valacyclovir hydrochloride to valacyclovir hydrochloride for IV.

In yet another aspect, the present invention relates to valacyclovir hydrochloride form IV, characterized by x-ray diffraction peaks at about 3.6, 10.7, 15.1, 26.9, and 28.1 ± 0.2°.

Valacyclovir hydrochloride form IV can be characterized by reflections in x-ray diffraction peaks about 3.6°, 10.7°, 15.1@ , 26.9°, and 28.1° ± 0.2 two-theta.

Valacyclovir hydrochloride forms I and IV are described in published United States Patent Application 2003/0114470 and published International Patent Application WO 2003/022209.

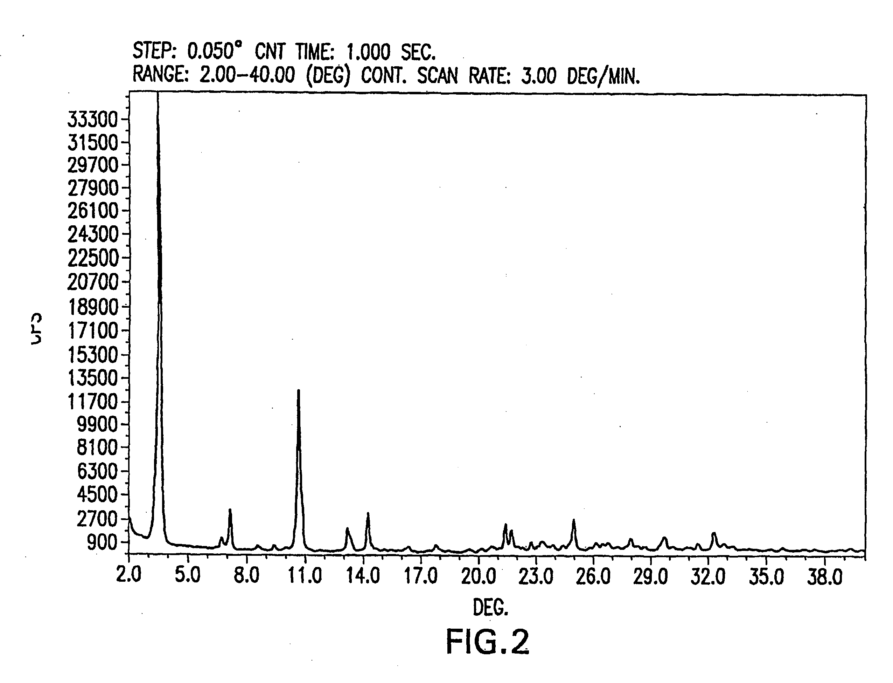

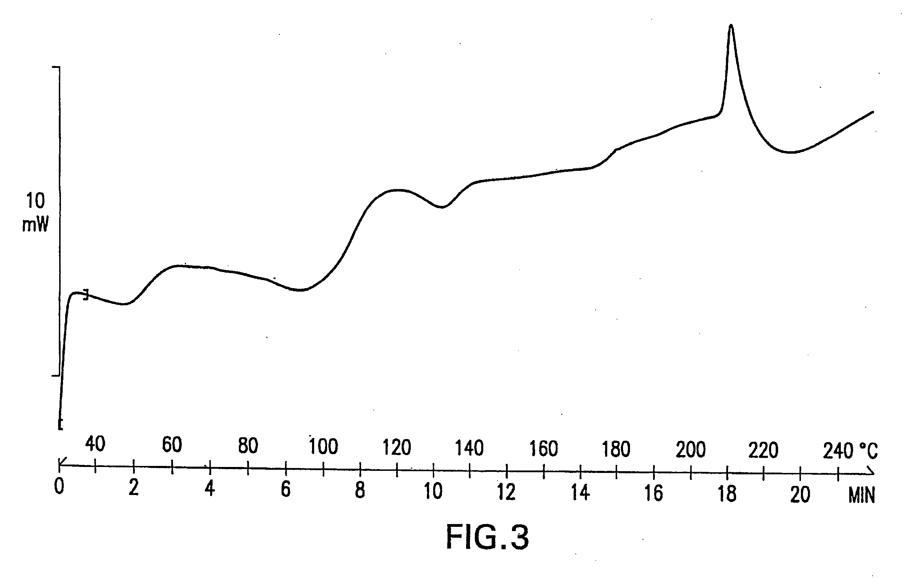

In another embodiment, the present invention provides a novel crystalline form of valacyclovir hydrochloride, denominated form VIII. Valacyclovir hydrochloride in form VIII can be characterized by either reflections in x-ray diffraction at about 7.1°, 10.7°, 13.2°, 14.2°, 21.4°, 21.8°, and 25.0° ± 0.2° 2θ, or by DSC endotherms at about 60°, 100°, and 133°C. A typical x-ray diffraction diagram for form VIII is given in Figure 2. A typical DSC thermogram for form VIII is given in Figure 3.

Valacyclovir hydrochloride in form VIII exhibits a loss on drying in TGA of about 20% to about 60%. Drying form VIII at 20° to 120°C for at least about 30 min yields a hydrate of valacyclovir hydrochloride form I.

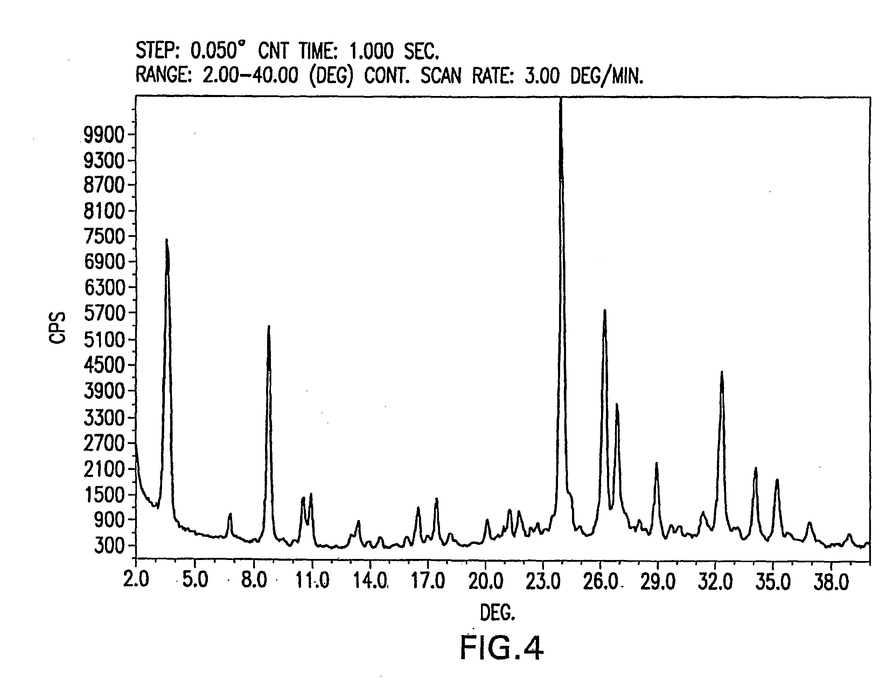

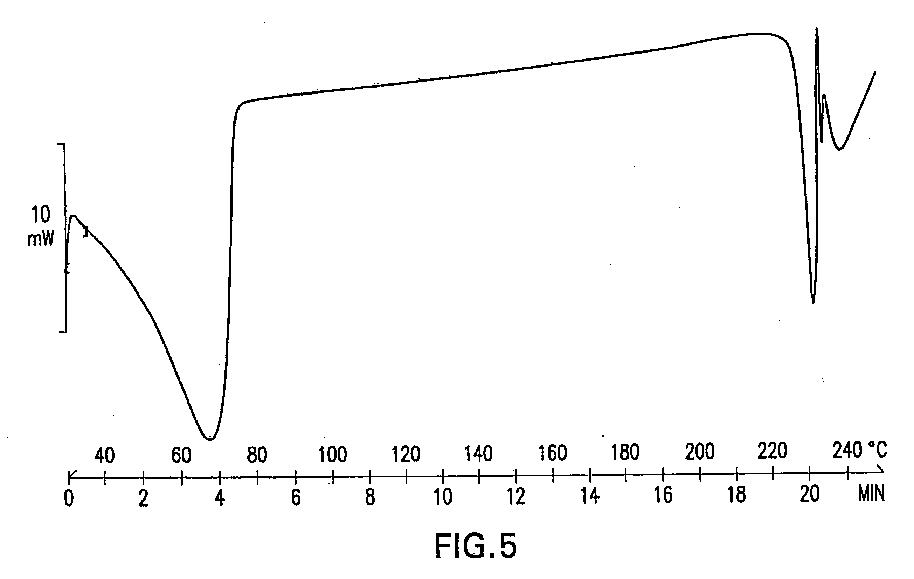

In another embodiment, the present invention provides a novel crystalline form of valacyclovir hydrochloride, denominated form IX. Valacyclovir hydrochloride form IX can be characterized by either reflections in x-ray diffraction at 8.7°, 10.5°, 24.1°, 26.3°, and 27.0° ± 0.3° 2θ, or by a DSC endotherm at about 67° C. A typical x-ray diffraction diagram of form IX is shown in Figure 4. A typical DSC thermogram of form IX is shown in Figure 5.

Valacyclovir hydrochloride form IX exhibits a loss on drying in TGA of about 20% to about 50%. Drying form IX at 50° to 60° C for at least about 30 min yields an anhydrous form of valacyclovir hydrochloride. *See, e.g.,* United States Patent 6,107,302.

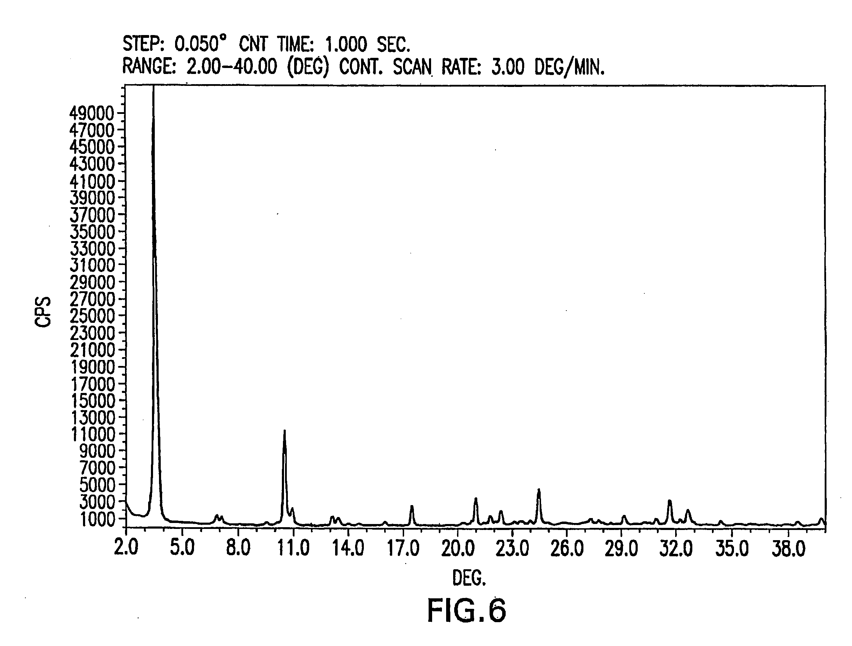

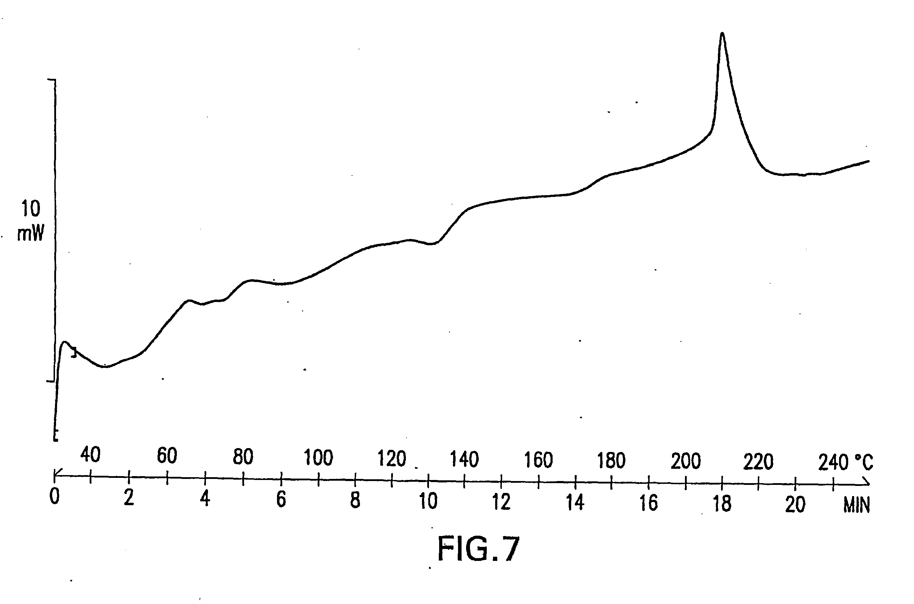

In a further embodiment, the present invention provides a novel crystalline form of valacyclovir hydrochloride, denominated form X. Valacyclovir hydrochloride form X can be characterized by either reflections in x-ray diffraction at about 6.7°, 7.0°, 17.5°, 21.0°, 22.4°, 24.5°, and 31.6° ± 0.2° 2θ, or DSC endotherms at about 55°, 75°, 95°, and 133°C. A typical x-ray diffraction diagram for form X is given in Figure 6. A typical DSC thermogram of form X is given in Figure 7.

Form X exhibits a loss on drying in TGA of about 15% to about 35%. Drying form X at 50° to 60° C for about 30 min or more yields a hydrate of valacyclovir form I.

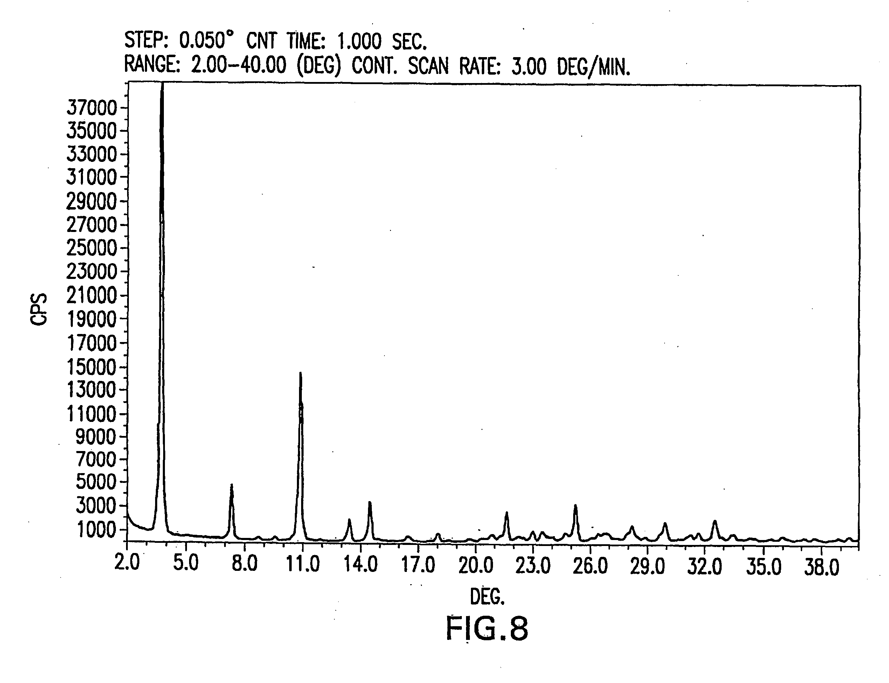

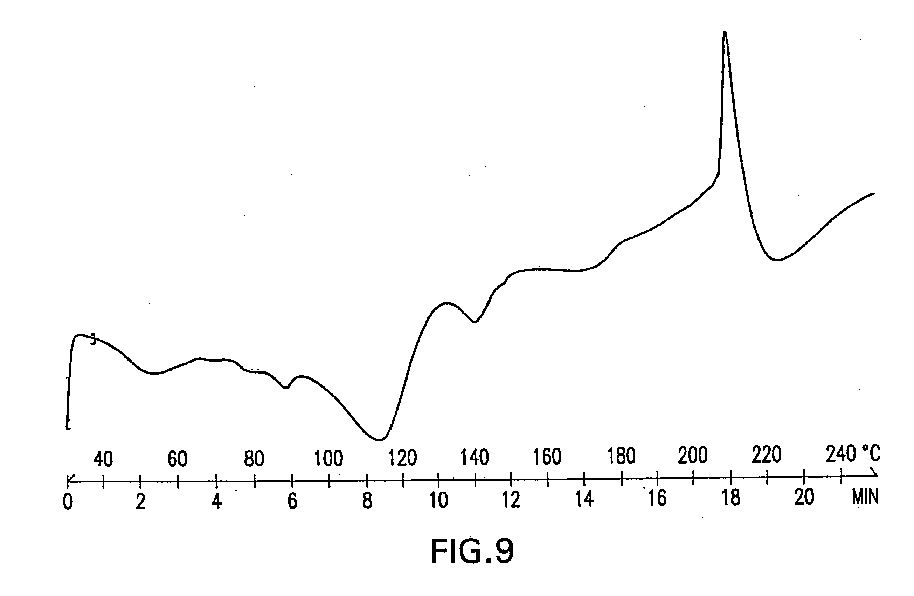

In a further embodiment, the present invention provides a novel crystalline form of valacyclovir hydrochloride, denominated form XI. Valacyclovir hydrochloride form XI can be characterized by either reflections in x-ray diffraction at about 14.4°, 21.6°, and 25.2°± 0.2° 2θ, or by DSC endotherms at about 49°, 107°, 132°, and 175°C. A typical x-ray diffraction diagram of form XI is shown in Figure 8. A typical DSC thermogram of form XI is shown in Figure 9.

Form XI exhibits a loss on drying in TGA of about 11% to about 13% in the temperature range of 25° to 140° C. The water content of form XI as determined by Karl Fisher analysis is about 6% to about 8%.

Reducing the water content of form XI to about 15 to 25% by, for example, drying results in transformation to form IV. When drying is used, the drying can be at a temperature of about 30° to about 50°C.

In yet another embodiment, the present invention provides a novel crystalline form of valacyclovir hydrochloride, denominated form XII. Form XII can be characterized by either x-ray reflections at 7.2°, 11.9°, 27.4°, and 28.1° ± 0.2° 2θ, or by a DSC thermogram that exhibits a double peak with minima at about 135° and 155° C, with a peak at about 177° C.

Form XII exhibits a loss on drying in TGA of about 9% in the temperature range of 25° to 180° C. The water content of form XII as determined by Karl Fisher analysis is about 9%.

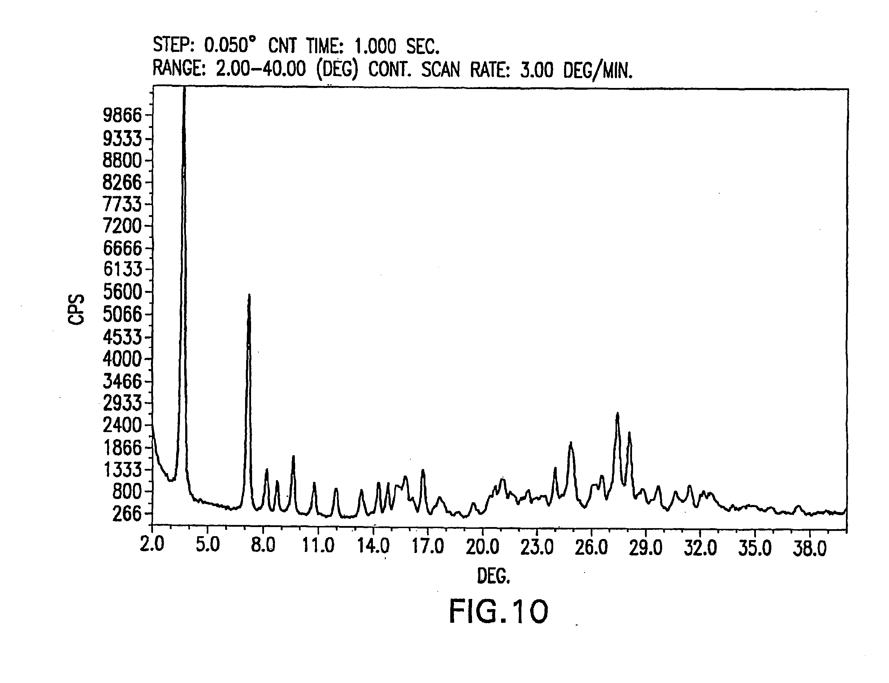

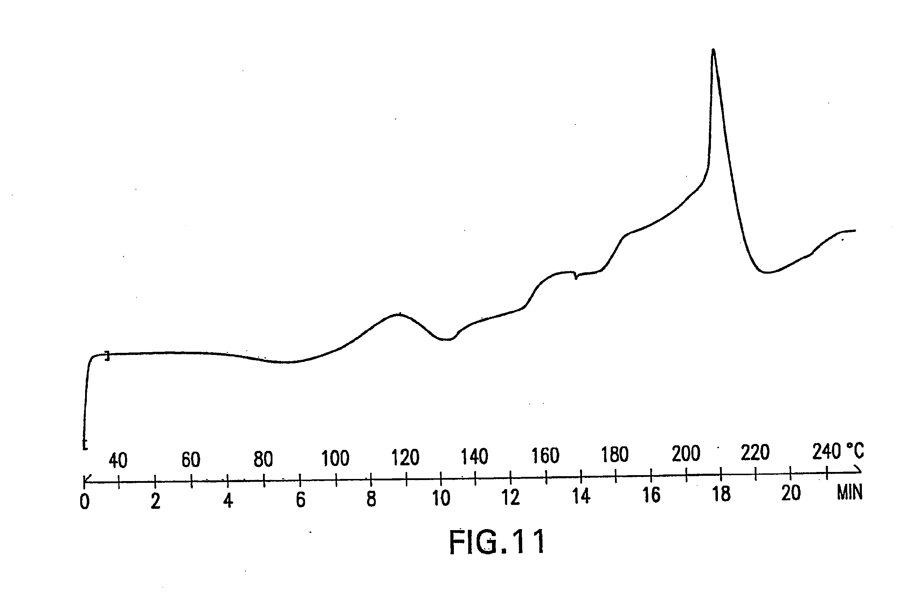

A typical x-ray diffraction diagram of form XII is given in Figure 10. A typical DSC thermogram of form XII is given in Figure 11.

Exposing form XII to an atmosphere of about 50% relative humidity for a day or more, preferably more, most preferably 3 or more days, results in transformation to valacyclovir hydrochloride form I.

The novel crystalline forms (polymorphs and pseudopolymorphs) of valacyclovir hydrochloride of the present invention can be prepared by any one or more of the methods described below, each of which represents an embodiment of the present invention. Two methods used in particular embodiments are: (1) the crystallization method; and (2), the precipitation method.

In certain embodiments, the crystal forms of the present invention can be made by a precipitation method that includes the step of mixing, with mechanical agitation, a solution of valacyclovir hydrochloride in a first solvent with a second solvent to form a suspension. Preferably, valacyclovir hydrochloride is practically insoluble in the second solvent. The first solvent can be a solvent for valacyclovir hydrochloride, or it can be a mixture of a solvent for valacyclovir hydrochloride (e.g. water) and a non-solvent, or anti-solvent, for valacyclovir hydrochloride (e.g. iso-propanol (IPA)).

An anti-solvent is an organic compound, normally a liquid at ambient temperature, that is a poor solvent or nonsolvent for the compound to be crystallized (here valacyclovir hydrochloride). The solubility of the compound to be crystallized from the combination of solvent and anti-solvent is lower than the solubility of the compound in the original solvent. In particular embodiments, crystallization is induced through use of an anti-solvent and by lowering the temperature of the solution.

The concentration of valacyclovir hydrochloride in first solvent can vary from between about 5% to about 30 % (w/v). The ratio of the volume of second solvent to solution varies depending on the composition of the first solvent, the concentration of the solution, and the temperature.

Mechanical agitation can be provided by any means known in the art, for example magnetic stirrers or paddle-, propeller- or turbine-type stirrers, to mention just a few. The skilled artisan will know to select the means of agitation depending on, among other things, the size and geometry of the vessel being used and the viscosity of the solution and suspension.

In preferred embodiments that incorporate the precipitation method, the method can include the step of agitating the suspension for about 2 to about 24 hours at a temperature of about -5° C or less.

Valacyclovir hydrochloride in the resulting crystal form can be isolated from the suspension by any means known in the art. For example, filtration (gravity or suction) or centrifugation can be used, to mention just two. After isolation, the valacyclovir hydrochloride in the resulting crystal form can be dried at atmospheric pressure or at reduced pressure (vacuum), both methods of which are known in the art.

It will be understood by those of skill in the art that other methods may also be used to produce the crystalline forms disclosed herein.

Water and water/IPA are the preferred first solvent. The preferred second solvent is IPA.

In another embodiment, the present invention provides a method of making valacyclovir hydrochloride in form V by the precipitation method, for example by mixing a solution of valacyclovir hydrochloride in a first solvent with a second solvent that can be an alcohol, preferably isopropanol, or acetone.

The solution is in a first solvent that includes water and, optionally, a water-miscible organic solvent such as acetic acid, a water-miscible ketone, or, preferably, an alcohol. When a ketone is used, acetone is the preferred ketone. When alcohol is used, isopropanol is the preferred alcohol. Preferably, water is the major constituent of the first solvent. Most preferably, the first solvent is water.

Preferably, the solution in the first solvent contains one part by weight valacyclovir hydrochloride and about 2 to about 6 parts by weight solvent. The solution can be made by, for example, dissolving the desired amount of valacyclovir hydrochloride in about 2 to about 6 parts by weight solvent. The valacyclovir hydrochloride can be made by any means known in the art, or it can be generated *in situ* from t-butoxycarbonyl valacyclovir (t-BOC Val), in which the nitrogen of the valine residue attached to the acyclovir moiety bears a t-butoxycarbonyl group.

The valacyclovir hydrochloride can be provided from any spource, or it can be generated *in situ.* When valacyclovir hydrochloride is generated *in situ* in a preferred embodiment, about 3 to about 7, preferably about 5, equivalents of hydrogen chloride, dissolved in a suitable vehicle, are added, preferably slowly to maintain temperature control, to a suspension of a protected valacyclovir (e.g., t-BOC valacyclovir) in a suitable solvent mentioned above. The vehicle can be any of those solvents mentioned above. Preferably, the vehicle and solvent are both water.

After addition of the hydrogen chloride, the mixture is stirred at a temperature below about 40° C, preferably at about 20° to 25° C, until the mixture essentially becomes a solution that can be roily or turbid. The mixture is then cooled to a temperature below about 10° C, preferably at about 0° C, and mixed with an alcohol, preferably isopropanol (20 to 30 volumes based on the volume of solvent used) to form a suspension. Preferably, the suspension is stirred for at least about one-half hour at this temperature.

Valacyclovir hydrochloride in form V can be isolated from the suspension by any means known in the art. For example, isolation can be by filtration (gravity or suction) or by centrifugation, to mention just two.

Form V can also be prepared by a precipitation method wherein an aqueous solution (20% to 30%, w/v) of valacyclovir hydrochloride is combined with acetone at about 40°C. The combination is then stirred at -15°C and form V is isolated from the resulting suspension in the usual way.

Typically, valacyclovir hydrochloride in form V prepared as described above will have a chemical purity of at least about 97%.

In a further embodiment, the present invention provides a crystallization modification of the precipitation method for making form V including the steps of providing, at an initial temperature, a solution of valacyclovir in a mixed solvent including methanol and either water or acetone; cooling the solution to a second temperature, optionally maintaining the resulting mixture at or below the second temperature for a holding time, and isolating form V from the resulting suspension. When water is used, the ratio of water to methanol is about 1:15 and the initial temperature is reflux. When acetone is used, the ratio of acetone to methanol is about 1:3 and the initial temperature is about 54° C.

When water is used, the solution can be held at or near reflux for about 1 to about 3 hours, whereafter the slightly turbid solution is slowly cooled to 20°C and maintained at about 20°C for several hours, and valacyclovir hydrochloride is isolated from the resulting suspension.

When acetone is used the second temperature is -5°C and it is achieved at a cooling rate of about 60 degrees per hour. The resulting suspension can be held at the second temperature for a holding time. Valacyclovir hydrochloride form V is then isolated from the suspension in the usual way.

In still a further embodiment, the present invention provides a method of making valacyclovir hydrochloride form V including the steps of: A method of making valacyclovir hydrochloride form V comprising the steps of: providing a solution of valacyclovir hydrochloride in water; adding the obtained solution on acetone at a temperature of about 40°C; cooling the solution to -15°C; maintaining the suspention at - 15°C for a night; and isolating crystalline valacyclovir hydrochloride form V.

In still yet another embodiment, the present invention provides a method of making valacyclovir hydrochloride form V including the steps of: dissolving BOC-L-Valacyclovir in an acid, preferably an organic acid, most preferably formic acid formic acid; adding water and hydrochloric acid to the obtained solution; adding isopropanol; cooling the solution to a temperature of about 12°C or less; and isolating crystalline valacyclovir hydrochloride form V.

In another embodiment, the present invention provides a method of making valacyclovir hydrochloride form V that includes the steps of: providing an approximately 5% (w/v) solution of valacyclovir hydrochloride in a mixture of acetone and methanol, 1:3 (v:v) at a temperature of about 54° C; cooling the solution to about -5° C or less , at a cooling rate of about 10 degrees per hour; maintaining the resulting slurry at -5° C or less for a holding time; and isolating valacyclovir hydrochloride form V from the slurry.

In another embodiment, the present invention provides a precipitation method for making valacyclovir hydrochloride form VIII including the steps of providing a solution of valacyclovir hydrochloride in a mixture of water and iso-propanol (IPA), 50% to 60% v/v IPA, at an initial temperature between about 37°and 50°C preferably 37°- 38°C, combining the solution with IPA, optionally cooling to a first crystallization temperature, optionally seeding with valacyclovir hydrochloride, then cooling to a second crystallization temperature of about -5°C, and isolating valacyclovir hydrochloride form VIII.

The concentration of the solution is between about 14% and about 18% (w/v). When solution and IPA are combined at 37°to 38°C, the resulting mixture is cooled to about 35°C and, optionally, seeded. When seeded, the mixture is preferably maintained at the first crystallization temperature for about 3 to about 5 hours. The mixture is then cooled to -5°C and form VIII is isolated.

In a further embodiment, the present invention provides a crystallization modification of the precipitation method for making valacyclovir form VIII including the steps of providing a solution of valacyclovir in a mixture of water and IPA (ca. 50/50 v/v) at a temperature of about 37°to 38°C, cooling the solution to about 35° C, seeding the solution with valacyclovir hydrochloride , maintaining the seeded solution at 35°C for a holding time of about 3 to about 5 hours, cooling the resulting mixture to less than 0°C, preferably -5°C or below, and isolating valacyclovir hydrochloride form VIII.

In another embodiment, the present invention provides a crystallization method for making valacyclovir hydrochloride form IX including the steps of providing a solution of approximately 5% to 7% (w/v) in an approximately 1:5 v/v, mixture of water and IPA at a temperature of at least about 60° C, cooling the solution until turbidity is just observed (about 59° C), maintaining the mixture at this temperature for a first holding time of about 3 to about 5 hours, cooling the mixture to a second temperature, preferably - 5°C, at about 5 degrees per hour, and, optionally, maintaining the resulting suspension at 0°C or below for a second holding time, and isolating valacyclovir hydrochloride form IX.

In still a further embodiment, the present invention provides a precipitation method for making valacyclovir hydrochloride form X including the steps of providing an aqueous solution of valacyclovir hydrochloride (ca. 30%, w/v) at 32°C, combining the solution with sufficient ethanol to render the solution turbid, maintaining the combination at 32°C for a holding time, preferably 1 to 3 hours, combining a second volume of ethanol with the combination (volume approximately equal to the first volume of ethanol initially added), cooling the a temperature less than about 0° C, preferably -5°C or less, at a cooling rate of about 5 degrees per hour, optionally maintaining the resulting suspension at the lower temperature for a second holding time, and isolating form X from the suspension.

In another embodiment, the present invention provides a crystallization method for making valacyclovir hydrochloride form XI including the steps of rapidly cooling a solution (*ca.* 15% w/v) of valacyclovir hydrochloride in a 1:3, v/v, mixture of water and IPA, initially at a temperature > 55°C, to about 55°C, cooling the resulting suspension to 40°C at about 1 to about 2 degrees per hour, optionally maintaining the resulting slurry, with stirring, at 40° C for a holding time, and isolating valacyclovir hydrochloride form XI.

In a further embodiment, the present invention provides a precipitation method for making valacyclovir hydrochloride form XI including the steps of adding, dropwise, cooled (*ca.* -5° C) IPA to an aqueous solution of valacyclovir hydrochloride (ca. 30%, w/v) untill massive precipitation occurs (*ca.* 2 volumes of cooled IPA per volume of aqueous solution), cooling the suspension to -5°C, and isolating crystalline valacyclovir hydrochloride form XI.

In yet another embodiment, the present invention provides a precipitation method for making valacyclovir hydrochloride in crystalline form XII including the steps of: providing an aqueous solution (ca. 25%, w/v) of valacyclovir hydrochloride at about 40° C, adding portionwise, in small aliquots, preferably dropwise, 3 to 4 volumes (per volume of solution) of IPA, cooling the resulting suspension to -5°C, and isolating crystalline valacyclovir hydrochloride form XII from the resulting suspension.

### Methods of use, Formulations, dosages

Valacyclovir hydrochloride may be formulated into a variety of pharmaceutical compositions and dosage forms that are useful in treating patients afflicted with viral infections, particularly infections caused by the herpes group of viruses.

In one embodiment, the present invention relates to pharmaceutical compositions including valacyclovir hydrochloride in at least one of forms XIII, IX, X, XI, and XII. In addition to the active ingredient(s), valacyclovir hydrochloride pharmaceutical compositions of the present invention may contain one or more excipients. Excipients are added to the composition for a variety of purposes.

Diluents increase the bulk of a solid pharmaceutical composition and may make a pharmaceutical dosage form containing the composition easier for the patient and caregiver to handle. Diluents for solid compositions include, for example, microcrystalline cellulose (e.g. AVICEL^{®}, microfine cellulose, lactose, starch, pregelitinized starch, calcium carbonate, calcium sulfate, sugar, dextrates, dextrin, dextrose, dibasic calcium phosphate dihydrate, tribasic calcium phosphate, kaolin, magnesium carbonate, magnesium oxide, maltodextrin, mannitol, polymethacrylates (e.g. Eudragit^{®}), potassium chloride, powdered cellulose, sodium chloride, sorbitol and talc.

Solid pharmaceutical compositions that are compacted into a dosage form like a tablet may include excipients whose functions include helping to bind the active ingredient and other excipients together after compression. Binders for solid pharmaceutical compositions include acacia, alginic acid, carbomer (e.g. carbopol), carboxymethylcellulose sodium, dextrin, ethyl cellulose, gelatin, guar gum, hydrogenated vegetable oil, hydroxyethyl cellulose, hydroxypropyl cellulose (e.g. KLUCEL^{®}), hydroxypropyl methyl cellulose (e.g. METHOCEL^{®}), liquid glucose, magnesium aluminum silicate, maltodextrin, methylcellulose, polymethacrylates, povidone (e.g. KOLLIDON^{®}, PLASDONE^{®}), pregelatinized starch, sodium alginate and starch.

The dissolution rate of a compacted solid pharmaceutical composition in the patient's stomach may be increased by the addition of a disintegrant to the composition. Disintegrants include alginic acid, carboxymethylcellulose calcium, carboxymethylcellulose sodium (e.g. Ac-DI-SOL^{®}, PRIMELLOSE^{®}), colloidal silicon dioxide, croscarmellose sodium, crospovidone (e.g. KOLLIDON^{®}, POLYPLASDONE^{®}), guar gum, magnesium aluminum silicate, methyl cellulose, microcrystalline cellulose, polacrilin potassium, powdered cellulose, pregelatinized starch, sodium alginate, sodium starch glycolate (e.g. EXPLOTAB^{®}) and starch.

Glidants can be added to improve the flow properties of non-compacted solid compositions and improve the accuracy of dosing. Excipients that may function as glidants include colloidal silicon dixoide, magnesium trisilicate, powdered cellulose, starch, talc and tribasic calcium phosphate.

When a dosage form such as a tablet is made by compaction of a powdered composition, the composition is subjected to pressure from a punch and dye. Some excipients and active ingredients have a tendency to adhere to the surfaces of the punch and dye, which can cause the product to have pitting and other surface irregularities. A lubricant can be added to the composition to reduce adhesion and ease release of the product from the dye. Lubricants include magnesium stearate, calcium stearate, glyceryl monostearate, glyceryl palmitostearate, hydrogenated castor oil, hydrogenated vegetable oil, mineral oil, polyethylene glycol, sodium benzoate, sodium lauryl sulfate, sodium stearyl famarate, stearic acid, talc and zinc stearate.

Flavoring agents and flavor enhancers make the dosage form more palatable to the patient. Common flavoring agents and flavor enhancers for pharmaceutical products that may be included in the composition of the present invention include maltol, vanillin, ethyl vanillin, menthol, citric acid, fumaric acid ethyl maltol, and tartaric acid.

Compositions may also be colored using any pharmaceutically acceptable colorant to improve their appearance and/or facilitate patient identification of the product and unit dosage level.

Selection of excipients and the amounts to use may be readily determined by the formulation scientist based upon experience and consideration of standard procedures and reference works in the field.

The solid compositions of the present invention include powders, granulates, aggregates and compacted compositions. The dosages include dosages suitable for oral, buccal, rectal, parenteral (including subcutaneous, intramuscular, and intravenous), inhalant and ophthalmic administration. Although the most suitable route in any given case will depend on the nature and severity of the condition being treated, the most preferred route of the present invention is oral. The dosages may be conveniently presented in unit dosage form and prepared by any of the methods well-known in the pharmaceutical arts.

Dosage formes include solid dosage forms like tablets, powders, capsules, suppositories, sachets, troches and lozenges as well as liquid syrups, suspensions and elixirs. An especially preferred dosage form of the present invention is a tablet.

The currently marketed form of valacyclovir (VALTREX^{®}) contains valacyclovir hydrochloride equivalent to 500 mg valacyclovir and the inactive ingredients carnauba wax, colloidal silicon dioxide crospovidone, FD&C Blue No. 2 Lake, hydroxypropyl methylcellulose, magnesium stearate, microcrystalline cellulose polyethylene glycol, polysorbate 80, povidone and titanium dioxide.

The function and advantage of these and other embodiments of the present invention will be more fully understood from the examples below. The following examples demonstrate the preparation of various crystalline forms of valacyclovir hydrochloride by the slurry method (examples 1 to 21), the vapor incubation method (examples 22 to 27), and the precipitation method (examples 28 to 32). Preparation of form I by the heating and evaporative method are also illustrated in examples 33 and 34 respectively. These examples are intended to illustrate the benefits of the present invention, but are not intended to limit the scope of the invention.

### EXAMPLES

### Example 1

Valacyclovir hydrochloride (8.8 g) was dissolved in water (35.2 g) at 40°C. The resulting solution was dropped into warm acetone (40°C). The resulting suspension was stirred at 25°C for 1 hour, then cooled to -15C and stirred overnight. The suspension was filtered and washed with cold (0°C) acetone. The material so obtained was dried to a constant weight at 25°C at reduced pressure. The material was form V according XRD.

### Example 2

Valacyclovir hydrochloride (39 g) was dissolved in a mixture of 10 g water and 150 mL methanol at reflux (*ca.* 65° C). The combination was stirred at reflux for 2 hours. Slight turbidity was observed. The combination was stirred for an additional 2 hours and then cooled to 20°C over 5 hours, stirred for 3 hours at 20° C, and filtered. The solid obtained (17.7 g) was found by XRD to be valacyclovir hydrochloride form V.

### Example 3

Valacyclovir hydrochloride (30 g) was dissolved in a mixture of 150 mLacetone and 450 mL methanol at 54° C. The solution was cooled to minus 5°C over 6 hours and the resulting suspension stirred at -5°C for 60 hours. The suspension was filtered and the solid collected and dried at 50°C under vacuum. The product, both before and after drying, was identified by XRD as valacyclovir hydrochloride form V.

### Example 4

BOC-L-Valacyclovir (6 kg) is dissolved in 18 kg of formic acid at 15-30 degrees. Water (1.9 kg) and HCl solution (3.2 kg, about 32% in water) was added. The solution was stirred for about 4 hours. IPA (7.5 kg) was added at 15-25°C to the solution. Precipitation is occurred. The mixture was cooled to temperature lower than 12 degrees and stirred for 2 hours at temperature lower than 12 degrees. The solids were filtered. The resulting wet substance is valacyclovir form V. The dried substance obtained is form V.

### Example 5

Valacyclovir HCl (8.8 g) is dissolved in 35.2 g of water at 40 C. The resulting solution is dropped onto warm acetone (40 C). The obtained suspension was stirred at 25 C for 1 h, then cooled to -15 C and stirred overnight. The suspension was filtered and washed with cold (0° C) acetone (20 g). The resulting substance was dried under reduced pressure at 25 C to constant weight to give valacyclovir HCl form V.

### Example 6

Valacyclovir HCl (39 g) was dissolved in solution of 10 g of water and 150 mL of methanol at reflux (~65 C), and stirred at reflux for 2 h until slight turbidity was obtained. The turbid solution was stirred for an additional 2 h and then cooled to 20° C over 5 h, stirred for 3 h at 20° C and filtered. The resulting isolated wet substance (17.7 g) is valacyclovir HCl form V.

### Example 7

Valacyclovir HCl (30 g)was dissolved in mixture of 150 mLof acetone and 450 mL of methanol at 54 C. The solution was cooled to -5° C over 6 hours and stirred at -5° C for 60 hours. The precipitated substance was collected by filtration and dried at 50 C in vacuum. The resulting wet and dry substance was identified by x-ray as form V.

### Example 8

Valacyclovir hydrochloride (30 g) was dissolved in a mixture of 90 mL water and 110 mL IPA at 50° C. IPA (190 ml) was added slowly until slight turbidity was observed. The mixture was stirred for 2 hours and then 190 mL of IPA were added, drop wise, at 50° C. Massive precipitation was observed. The resulting mixture was cooled to -5°C and then filtered. The material collected was found by XRD to be valacyclovir hydrochloride form VIII.

Drying of form VIII at 50-120°C resulted in form I.

### Example 9

Valacyclovir hydrochloride (30 g) was dissolved in a mixture of 90 mL water and 90 mL IPA at 37°-38° C. IPA 25 mL was added and slight turbidity was observed. Additional IPA (395 ml) was added at the same temperature. The resulting mixture was cooled to -5°C and filtered. The wet material was analyzed by XRD and found to be valacyclovir hydrochloride form VIII.

### Example 10

Valacyclovir hydrochloride (30 g) was dissolved in a mixture of 90 mL water and 90 mL of IPA at 37°-38° C. The mixture was cooled to 35° C, seeded with valacyclovir hydrochloride, stirred for 4 hours at 35° C, and cooled to -5°C over 12 hours. The resulting suspension was filtered and the solid collected analyzed wet by XRD and found to be valacyclovir hydrochloride form VIII.

### Example 11

Valacyclovir hydrochloride (30 g) was dissolved in a mixture of 90 mL water and 420 mL IPA at 61° C. The solution was cooled to 58° C, seeded with valacyclovir hydrochloride, and stirred 2 hours at 58° C. The mixture was then cooled to -5°C and the resulting suspension filtered. The solid collected was analyzed wet by XRD and found to be valacyclovir hydrochloride form VIII.

### Example 12

Valacyclovir hydrochloride (30 g) was dissolved in a mixture of 90 mL water and 30 mLIPA at 35° C. The solution was cooled to 10° C. The resulting suspension was filtered and the collected solid analyzed wet by XRD and found to be valacyclovir hydrochloride form VIII.

### Example 13

Valacyclovir hydrochloride (30 g) was dissolved in a mixture of 90 mL water and 420 mLIPA at 62° C. The resulting solution was cooled to 59°C and slight turbidity was observed. The mixture was stirred for 4 hours at 59°C and then cooled to -5°C over 12 hours. The resulting mixture was stirred at -5°C for 3 hours, filtered, and washed width IPA. The wet solid was analyzed by XRD and found to be valacyclovir hydrochloride form IX.

Drying of form IX led to a mixture of form I and anhydrous form.

### Example 14

Valacyclovir hydrochloride (30 g) was dissolved in 90 mL of water at 32° C. Absolute ethanol (210 ml) was added drop wise into the solution and slight turbidity was observed. The mixture was stirred for 2 hours at 32°C and an additional 210 mL of ethanol was added to the mixture. The resulting mixture was cooled to -5°C over 5 hours and maintained at -5° C, with stirring, for 12 hours. The suspension was filtered and the solid collected was analyzed by XRD and found to be valacyclovir hydrochloride form X.

### Example 15

Valacyclovir hydro,chloride (30 g) was dissolved in a mixture of 50 g water and 150 mLIPA at about 80°C (reflux). The solution was ballistically cooled to 55°C and massive precipitation began. The resulting mixture was cooled to 40°C for 1 hour, stirred for one-half hour, and filtered. The solid collected was analyzed wet and found by XRD to be valacyclovir hydrochloride form XI.

Upon drying, this product transformed to form I.

### Example 16

Valacyclovir hydrochloride was dissolved in water (90 g) at 37° C. Cooled (-5° C) IPA (150 ml) was added dropwise into the resulting solution and massive precipitation occurred. The resulting mixture was cooled to -5°C and filtered. The resulting substance (wet) was identified as valacyclovir hydrochloride form XI. Upon drying, the material converted to form I.

### Example 17

Valacyclovir hydrochloride (30 g) and water (120 g) were charged to a 0.5 L reactor. The mixture was heated to 40°C to effect dissolution. The solution was stirred at 50 rpm. IPA (420 ml) was added, drop wise, over 4 hours at 40° C. The mixture was stirred at 40°C for 60 minutes, then cooled to -5°C over 12 hours. The product was collected by suction filtration and washed with IPA. The wet product was dried overnight *in vacuo* (35-40 mm hg) at 50° C. The product was analyzed by XRD and found to be valacyclovir hydrochloride form XII.

### Example 18

Valacyclovir hydrochloride (14 g) and water (56 g) were charged to a 0.25 L jacketed reactor. The mixture was heated to 40°C to effect dissolution and stirred at 300 rpm. IPA (196 ml) was added, drop wise, over 0.5 hours at 40° C. The mixture was stirred at 40°C for 60 minutes and cooled to -5°C over 3 hours. The product was collected by section filtration and washed with cold IPA. The wet product was dried at 50°C *in vacuo* (35-40 mm Hg). The dried product was analyzed by XRD and found to be a valacyclovir hydrochloride form XII.

### Example 19

Valacyclovir hydrochloride (14 g), formic acid (2ml) and water (56 g) were charged to a 0.25 L jacketed reactor. The mixture was heated to 40°C to effect dissolution and stirred at 30 rpm. IPA (196 ml) was added, dropwise, over 0.5 hours at 40° C. The mixture was stirred at 40°C for 60 minutes. The mixture was cooled to -5° over 3 hours. The product was collected by suction filtration and washed with cold IPA. The product was dried at 50°C overnight *in vacuo* (35-40 mm Hg). XRD analysis showed the product to be form XII.

### Example 20

| Raw materials: | |
|---|---|
| Valacyclovir(crude dry) | 20g |
| Formic acid | 20 ml |
| Water | 10 ml |
| IPA | 100 ml |

### Procedure:

Twenty grartis of valacyclovir (crude dry), 20 ml of formic acid, and 10 mL of process water were charged into a jacketed 0.25L reactor. The contents of the reactor were heated to 40 degrees (jacket temperature) to dissolution. One hundred mL of IPA were added dropwise during 1 hour at 40 degrees. The contents of the reactor were stirred at 40 degrees for 60 min and cooled to -7°C (jacket temperature) during 3 hours. The product was collected (isolated) by suction filtration. The wet cake was washed with 20 mL of cold IPA.

The wet product was dried in a vacuum oven at 50° C and about 35 - 40 mm Hg overnight (about 12-15 hours). The isolated material was analyzed and found to be form V.

### Example 21

Forty kg of Valacyclovir are dissolved in 160 L of water at 35 degrees. Six hundred liters (600 L) of IPA are added to the solution. Precipitation occurs. The resulting slurry is cooled to about 10 degrees over about 4 hours and stirred for about 2 hours at about 10° C. The solids are collected on a centrifuge to a moisture content of about 55% in the filtered cake. The isolated substance is form XI.

### Example 22

Forty kg of valacyclovir are dissolved in 160 L of water at 35 degrees. Six hundred L of IPA are added to the solution. Precipitation occurs. The resulting slurry is cooled to about 10°C over about 4 hours and stirred for about 2 hours at about 10°C. The solids are collected on a centrifuge to moisture content of about 55% of the filtered cake. The substance obtained is form XI. The wet substance is drying on the vacuum of 250-350 mm Hg at about 35 degrees up to humidity of about 20%. The resulting substance is form IV.

### Example 23

Forty kg of valacyclovir are dissolved in 160 L of water at 35 degrees. Six hundred L of IPA are adding to the solution. Precipitation occurs. The slurry is cooled to about 10 degrees for about 4 hours and stirred for about 2 hours at about 10°C. The solids are collected on a centrifuge to a moisture content of about 55% in the filtered cake. The substance obtained is form XI. The wet sample is squeezed on a Buchner filter up to humidity of about 20%. The resulting obtained substance is form VIII.

### Example 24

Forty kg of valacyclovir are dissolved in 160 L of water at 35 degrees. Six hundred (600) L of IPA were adding to the solution. Precipitation occurred. The resulting slurry is cooled to about 10°C degrees over about 4 hours and stirred for about 2 hours at about 10° C. The solids are collected on a centrifuge to moisture of about 55% in the filtered cake. The substance obtained is form XI. The wet sample is dried under pressure of about 700 mm Hg and at about 35°C degrees. The sample after 16 hours was checked and about 35% humidity form VIII was found. After 20 hours of drying the sample checked was found as of 30% humidity and of form IV.

Further aspects and features of the present inventions are set out in the following numbered clauses.
1. A crystalline form of valacyclovir hydrochloride having at least one characteristic selected from:
   a) x-ray diffraction reflections at 7.1°, 10.7°, 13.2°, 14.2°, 21.4°, 21.8°, and 25.0° ± 0.2° 2θ, and
   b) DSC endotherms at about 60°C, 100°C, and 133°C.
2. The crystalline form of valacyclovir hydrochloride of clause 1 characterized by x-ray diffraction reflections at 7.1°, 10.7°, 13.2°, 14.2°, 21.4°, 21.8°, and 25,0° ± 0.2° 28.
3. The crystalline form of valacyclovir hydrochloride of clause 2 having a powder x-ray diffraction diagram substantially as shown in Figure 2.
4. The crystalline form of valacyclovir hydrochloride of clause 1 having DSC endotherms at about 60°C, 100°C, and 133°C.
5. The crystalline valacyclovir hydrochloride of clause 4 having a DSC thermogram substantially as shown in Figure 3.
6. A method of making the crystalline form of valacyclovir hydrochloride of clause 1 comprising the steps of:
   a) providing a solution of at least about 15% (w/v) concentration of valacyclovir hydrochloride in a first solvent wherein the first solvent comprises at least about 50% (v/v) isopropanol, the remainder consisting essentially of water, at an initial temperature below about 60°C, and
   b) combining the solution with a second solvent consisting essentially of isopropanol.
7. The method of clause 6 wherein the first solvent comprises about 55% on a volume basis, isopropanol and the temperature at which the solution is provided is at least about 50° C.
8. The method of clause 7 wherein the volume of second solvent combined with the solution is provided at a temperature of about 50° C and the volume of second solvent combined with the solution.is equal to about twice the volume of the solution provided.
9. The method of clause 8 wherein the second solvent is added in first and second portions wherein the volumes of first portion and second portions are essentially equal to each other and essentially equal to the volume of solution provided.
10. The method of clause 6 further comprising the steps of cooling the combination of solution and second solvent to a temperature of about -5°C and isolating the crystalline form of valacyclovir hydrochloride.
11. The method of clause 6 wherein;
   the solution is provided at an initial temperature of about 37°C and a concentration of about 17% (w/v),
   the volume of the second solvent combined with the solution provided is about twice that of the solution provided, and
   the second solvent is added in first and second portions, whereby the volume of the first portion is just sufficient to render the solution turbid, where after the second portion is combined and the resulting combination is cooled to a temperature of about -5° C or less and the crystalline form of valacyclovir hydrochloride is isolated.
12. A method of making crystalline valacyclovir hydrochloride form I comprising the step of drying the crystalline form of clause 1, at a temperature of about 40 to about 90°C.
13. A method of making the crystalline form of valacyclovir hydrochloride of clause 1 comprising the steps of:
   a) providing a solution of valacyclovir hydrochloride in a crystallization solvent that is a mixture of first solvent that consists essentially of water and a second solvent that consists essentially of isopropanol at an initial temperature,
   b) cooling the solution provided to a seeding temperature,
   c) seeding the solution provided with valacyclovir hydrochloride,
   d) maintaining the seeded solution at the seeding temperature for a period of time from about 2 to about 4 hours, whereby a suspension is formed
   e) cooling the suspension to a temperature of about -5° C or less, and
   f) isolating the crystalline valacyclovir hydrochloride.
14. The method of clause 13 wherein;
   the crystallization solvent is a mixture of water and isopropanol, 50/50, v/v,
   the concentration of the solution provided is about 17%, w/v, and
   the initial temperature is about 37° C and the seeding temperature is about 35° C.
15. The method of clause 13 wherein;
   the solvent is a mixture of water and isopropanol, 18/82, v/v, the concentration of the solution provided is about 6%, w/v, and, the initial temperature is about 60°C and the seeding temperature is about 58°C.
16. A method of making the crystalline form of valacyclovir hydrochloride of clause 1 comprising the steps of:
   a) providing a solution of valacyclovir hydrochloride in a crystallization solvent that is a mixture of first solvent that consists essentially of water and a second solvent that consists essentially of isopropanol at an initial temperature,
   b) cooling the solution; and
   c) isolating the crystalline valacyclovir hydrochloride.
17. Crystalline valacyclovir hydrochloride prepared by the processes of any of clauses 6, 13 or 16.
18. Crystalline valacyclovir hydrochloride form VIII characterized by:
   a) x-ray diffraction reflections at 7.1°, 10.7°, 13.2°, 14.2°, 21.4°, 21.8°, and 25,0° ± 0.2° 2θ, and
   b) DSC endotherms at about 60°C, 100°C, and 133°C.
19. A crystalline form of valacyclovir hydrochloride having at least one characteristic selected from:
   a) x-ray diffraction reflections at 8.7° 10.5°, 24.1° 26.3° and 27.0° ± 0.2° 2θ, and
   b) a DSC endotherm with peak at about 67°C.
20. The crystalline valacyclovir hydrochloride of clause 19 characterized by x-ray diffraction reflections at 8.7° 10.5°, 24.1° 26.3° and 27.0° ± 0.2° 2θ.
21. The crystalline valacyclovir hydrochloride of clause 20 having a powder x-ray diffraction diagram substantially as shown in figure 4.
22. The crystalline valacyclovir hydrochloride of clause 19 having a DSC endotherm with peak at about 67°C.
23. The crystalline valacyclovir hydrochloride of clause 22 having a DSC thermogram substantially as shown in Figure 5.
24. A method of making the crystalline form of valacyclovir hydrochloride of clause 19 comprising the steps of:
   a) providing a solution of less than about 10% (w/v) concentration of valacyclovir hydrochloride in a solvent that is a 18/82 mixture, v/v, of a first solvent that consists essentially of water and a second solvent that consists essentially of isopropanol at an initial temperature of at least about 60°C,
   b) cooling the solution to a temperature of about 59°C and maintaining the solution at that temperature at least until the solution becomes turbid,
   c) cooling the turbid solution to a temperature of -5° C or below at a cooling rate of about 4 to about 6 degrees per minute, and
   d) isolating the crystalline valacyclovir hydrochloride.
25. Crystalline valacyclovir hydrochloride prepared by the process of clause 24.
26. Crystalline valacyclovir hydrochloride form IX characterized by:
   a) X-ray diffraction at 8.7° 10.5°, 24.1° 26.3° and 27.0° ±0.2° 20, and
   b) a DSC endotherm with peak at about 67°C.
27. A crystalline form of Valacyclovir hydrochloride having at least one characteristic selected from:
   a) x-ray diffraction reflections at 6.7°, 7.0°, 17.5°, 21.0°, 22.4°, 24.5°, and 31.6°:1: 0.2° 2θ, and
   b) DSC endotherms at about 55° C, 75° C, 95° C, and 133° C.
28. The crystalline form of valacyclovir hydrochloride of clause 27 characterized by x-ray diffraction reflections at 6.7°, 7.0°, 17.5°, 21.0°, 22.4°, 24.5°, and 31.6° ± 0.2° 2θ.
29. The crystalline valacyclovir hydrochloride of clause 28 having an x-ray diffraction diagram substantially as shown in Figure 6.
30. The crystalline valacyclovir hydrochloride of clause 27 characterized by DSC endotherms at about 55° C, 75° C, 95° C, and 133° C.
31. The crystalline form of valacyclovir hydrochloride of clause 30 having a DSC thermogram substantially as shown in figure 7.
32. A method of making the crystalline form of valacyclovir hydrochloride of clauses 27 comprising the steps of:
   a) providing, at a temperature of about 30°C to about 35°C, a solution of at least about 20% concentration, w/v, of valacyclovir hydrochloride in a first solvent that consists essentially of water,
   b) combining the solution with a first portion of second solvent, equal to about 2 to about 2.3 times the volume of solution, that consists essentially of ethanol, and maintaining the combination at a temperature of about 30°C to about 35°C for about 1 to about 3 hours,
   c) further combining the combination with a second portion of second solvent, equal to about 2 to about 2.3 times the initial volume of the solution provided,
   d) cooling the combination so obtained to a temperature of about -5°C or less, and
   e) isolating the crystalline valacyclovir hydrochloride.
33. A method of making crystalline valacyclovir hydrochloride form I by drying the crystalline form of clause 32 at a temperature of about 40°C to about 90°C.
34. Crystalline valacyclovir hydrochloride prepared by the process of clause 32.
35. Crystalline valacyclovir hydrochloride form X characterixed by:
   a) x-ray diffraction reflections at 6.7°, 7.0°, 17.5°, 21.0°, 22.4°, 24.5°, and 31.6°± 0.2° 2θ, and
   b) DSC endotherms at about 55° C, 75° C, 95° C, and 133° C.
36. A crystalline form of valacyclovir hydrochloride having at least one characteristic selected from:
   a) x-ray reflections at about 14.4°, 21.6°, and 25.2° ± 0.2° 2θ, and
   b) DSC endotherms at about 49°, 107°, 132°, and 175°C.
   c) water content of about 6% to about 8%, determined by Karl Fisher analysis.
37. The crystalline valacyclovir hydrochloride of clause 36 characterized by x-ray reflections at about 14.4°, 21.6°, and 25.2° ± 0.2° 2θ.
38. The crystalline valacyclovir hydrochloride of clause 37 having an x-ray diffraction diagram substantially as shown in Figure 8.
39. The crystalline valacyclovir hydrochloride of clause 36 characterized by DSC endotherms at about 49°, 107°, 132°, and 175°C.
40. The crystalline valacyclovir hydrochloride of clause 39 having a DSC thermogram substantially as shown in Figure 9.
41. A method of making crystalline form of valacyclovir hydrochloride of clause 36 comprising the steps of:
   a) providing, at a temperature of about 80°C, a solution of valacyclovir hydrochloride in a first solvent that consist essentially of water and a second solvent consisting essentially of isopropanol,
   b) rapidly cooling the solution provided to about 55°C,
   c) cooling the solution to a temperature of about 40°C; and
   d) isolating the crystalline valacyclovir hydrochloride.
42. A method of making the crystalline form of valacyclovir hydrochloride of clause 36 comprising the steps of:
   a) providing, at a temperature of about 37°C, a solution of valacyclovir hydrochloride in a first solvent that consist essentially of water,
   b) combinig the solution with a second solvent that consist essentially of isopropanol,
   c) cooling the solution so obtained to a temperature of about -5°C or less; and
   d) isolating the crystalline valacyclovir hydrochloride.
43. A method of making the crystalline valacyclovir hydrochloride form I comprising the step of drying the crystalline form of clause 36 at a temperature of about 40 to about 90°C.
44. A method of making the crystalline form of valacyclovir hydrochloride of clause 36 comprising the steps of:
   a) providing, at a temperature of about 30 to about 40°C, a solution of valacyclovir hydrochloride in a first solvent that consist essentially of water,
   b) combinig the solution with a second solvent that consist essentially of isopropanol,
   c) cooling the solution so obtained to a temperature of about 10°C or less;
   d) lowering the humidity to about 50-60%; and
   e) isolating the crystalline valacyclovir hydrochloride.
45. The method of clause 44, wherein the humidity lowering is performed by filtering.
46. A method of making crystalline valacyclovir hydrochloride form IV comprising the step of lowering the water content of the crystalline form of clause 36 to about 15-25%.
47. The method of clause 46, wherein the water content is lowered by drying at a temperature of about 30 to about 50°C.
48. A method of making the crystalline valacyclovir hydrochloride form VIII comprising the step of reducing the water content to about 15-25% of the crystalline form of clause 36.
49. The method of clause 48, wherein the reduction is performed by filtering under vacuum.
50. Valacycloivir hydrochloride form XI characterized by:
   a) x-ray reflections at about 14.4°, 21.6°, and 25.2° ± 0.2° 2θ, and
   b) DSC endotherms at about 49°, 107°, 132°, and 175°C.
   c) water content of about 6% to about 8%, determined by Karl Fisher.
51. Crystalline valacyclovir hydrochloride prepared by the process of any of clauses 41, 42, or 44.
52. A crystalline form of valacyclovir hydrochloride having at least one characteristic selected from:
   a) x-ray reflections at about 7.2°, 11.9°, 27.4°, and 28.1° ± 0.2° 2θ, and
   b) a multi-endothermic DSC thermogram having minima at about 135°and 155° C and a peak at about 177° C.
   c) water content of about 9%, determined by Karl Fisher analysis.
53. The crystalline valacyclovir hydrochloride of clause 52 characterized by x-ray reflections at about 7.2°, 11.9°, 27.4°, and 28.1° ± 0.2° 2θ.
54. The crystalline valacyclovir hydrochloride of clause 53 having an x-ray diffraction diagram substantially as shown in Figure 10.
55. The crystalline valacyclovir hydrochloride of clause 52 having a multi-endothermic DSC thermogram having minima at about 135° and 155° C and a peak at about 177° C.
56. The crystalline valacyclovir hydrochloride of clause 55 having a DSC thermogram substantially as shown in Figure 11.
57. A method of making the crystalline form of valacyclovir hydrochloride of clause 52 comprising the steps of:
   a) providing an aqueous solution of valacyclovir at about 40°C,
   b) adding, in small aliquots, preferably dropwise, 3 to 4 volumes (per volume of solution) of IPA,
   c) cooling the solution so obtained to a temperature of about -5°C or less; and
   d) isolating crystalline valacyclovir hydrochloride.
58. A method of making the crystalline form of valacyclovir hydrochloride of clause 52 comprising the steps of:
   a) providing a solution of valacyclovir in a first solvent that consist essentially of water and acid,
   b) adding a second solvent that consist essentially of isopropanol,
   c) cooling the solution so obtained to a temperature of about -5°C or less; and
   d) isolating crystalline valacyclovir hydrochloride.
59. The process of claim 58, wherein the acid is a formic acid.
60. A method of making crystalline valacyclovir hydrochloride form I by exposing to at least 50% of RH for at least about one day the crystalline form of clause 52.
61. Valacycloivir hydrochloride form XII characterized by:
   a) x-ray reflections at about 7.2°, 11.9°, 27.4°, and 28.1° ± 0.2° 2θ;
   b) multi-endothermic DSC thermogram having minima at about 135°and 155° C and a peak at about 177° C; and
   c) water content of about 9%, determined by Karl Fisher analysis.
62. Crystalline valacyclovir hydrochloride form XII prepared by the processes of either any of clauses 57 or 58.
63. A method of making valacyclovir hydrochloride form V comprising the steps of:
   a) providing, at reflux, an approximately 25% (w/v) solution of valacyclovir hydrochloride in a mixture of water and methanol, 1:15 (v:v);
   b) cooling the solution to 20° C at a cooling rate of about 10 degrees per hour;
   c) maintaining the resulting slurry at 20°C for a holding time; and
   d) isolating crystalline valacyclovir hydrochloride form V.
64. The method of clause 63 wherein the holding time is about 3 hours.
65. A method of making valacyclovir hydrochloride form V comprising the steps of:
   a) providing an approximately 5% (w/v) solution of valacyclovir hydrochloride in a mixture of acetone and methanol, 1:3 (v:v) at a temperature of about 54°C;
   b) cooling the solution to about -5° C or less , at a cooling rate of about 10 degrees per hour;
   c) maintaining the resulting slurry at -5 °C or less for a holding time; and
   d) isolating valacyclovir hydrochloride form V.
66. The method of clause 65 wherein the holding time is about 60 hours.
67. A method of making valacyclovir hydrochloride form V comprising the steps of:
   a) providing a solution of valacyclovir hydrochloride in water;
   b) adding the obtained solution on acetone at a temperature of about 40°C;
   c) cooling the solution to -15°C;
   d) maintaining the suspention at -15°C for a night; and
   e) isolating crystalline valacyclovir hydrochloride form V.
68. A method of making valacyclovir hydrochloride form V comprising the steps of:
   a) dissolving BOC-L-Valacyclovir in formic acid;
   b) adding water and hydrochloric acid to the obtained solution;
   c) adding isopropanol;
   d) cooling the solution to a temperature of about 12°C or less; and
   e) isolating crystalline valacyclovir hydrochloride form V.
69. A method of making valacyclovir hydrochloride form V comprising the steps of:
   a) providing a solution of valacyclovir in water and acid;
   b) heating the solution to a temperature of about 40°C;
   c) adding isopropanol;
   d) cooling the solution to a temperature of abot -5°C or less; and
   e) isolating crystalline valacyclovir hydrochloride form V.
70. Crystalline valacyclovir hydrochloride prepared by the processes of any of clauses 63, 65, 67, 68 or 69.
71. A pharmaceutical composition comprising one or more of the crystalline valacyclovir hydrochloride forms of any of clauses 1, 18, 19, 26, 27, 35, 36, 51, 52 62 or 70, and at least one pharmaceutically acceptable excipient.

## Claims

1. Valacycloivir hydrochloride **characterized by**:
a) x-ray reflections at about 14.4, 21.6, and 25.2±0.2 degrees 2-theta, or
b) DSC endotherms at about 49°C, 107°C, 132°C, and 175°C.

2. Valacycloivir hydrochloride according to claim 1 having a water content of about 6% to about 8% as determined by Karl Fisher analyais.

3. A method for making valacyclovir hydrochloride of claim 1 or claim 2 comprising (i) rapidly cooling a solution of valacyclovir hydrochloride in a 1:3 (v/v) mixture of water and IPA to a temperature of about 55°C, (ii) further cooling the resulting suspension to 40°C at about 1 to about 2 degrees per hour, and (iii) isolating valacyclovir hydrochloride.

4. A method for making valacyclovir hydrochloride of claim 1 or claim 2 comprising (i) adding, dropwise, cooled (-5°C) IPA to an aqueous solution of valacyclovir hydrochloride (ca. 30%, w/v) until massive precipitation occurs (ca. 2 volumes of cooled IPA per volume of aqueous solution), cooling the suspension to -5°C, and isolating crystalline valacyclovir hydrochloride.

5. A pharmaceutical composition comprising valacyclovir hydrochloride of claim 1 or claim 2 and at least one pharmaceutically acceptable excipient.

6. Valacyclovir hydrochloride of claim 1 or claim 2 for the treatment of viral infections, particulary herpes.
